(19) Europäisches Patentamt
European Patent Office
Office européen des brevets

(11) Publication number: **0 091 148**
**B1**

(12) # EUROPEAN PATENT SPECIFICATION

(45) Date of publication of patent specification: **19.11.87**

(51) Int. Cl.$^4$: **C 07 D 417/04,**
C 07 D 417/14, A 01 N 43/72

(21) Application number: **83200384.2**

(22) Date of filing: **21.03.83**

(54) **Fungicidal heterocyclic compounds.**

(30) Priority: **02.04.82 GB 8209786**

(43) Date of publication of application:
**12.10.83 Bulletin 83/41**

(45) Publication of the grant of the patent:
**19.11.87 Bulletin 87/47**

(84) Designated Contracting States:
**AT BE CH DE FR GB IT LI LU NL SE**

(56) References cited:
**EP-A-0 004 129**
**EP-A-0 010 420**
**FR-A-2 162 488**
**US-A-4 174 394**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Ten Haken, Pieter**
**Konkelboet The Street**
**Eastling Nr. Faversham Kent (GB)**
Inventor: **Webb, Shirley Beatrice**
**17 North Street Ashford Road**
**Sheldwich Faversham Kent (GB)**

(74) Representative: **Hunter, Keith Roger Ian et al**
**4 York Road**
**London SE1 7NA (GB)**

**EP 0 091 148 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to fungicidal heterocyclic compounds.

EP—A—4129 discloses certain pyridyl-substituted thiazolidinone derivatives having fungicidal activity.

EP—A—10420 also discloses certain pyridyl-substituted thiazolidine and 1,3-thiazine derivatives having fungicidal activity.

The present invention provides a compound of the general formula:

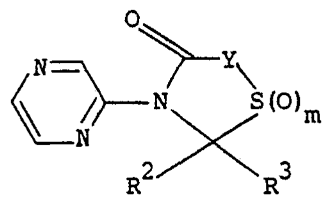

(I)

or a salt or a metal complex thereof, wherein Y represents a group —$CR^4R^5CR^6R^7$—; $R^2$ represents a phenyl group substituted by one or two halogen atoms; each of $R^3$, $R^4$, $R^5$, $R^6$ and $R^7$ independently represents a hydrogen atom or a $C_{(1-4)}$ alkyl group; and m is 0, 1 or 2.

Particularly suitable substituents for a phenyl ring represented by $R^2$ are bromine, or, preferably, chlorine or fluorine atoms.

Alkyl groups represented by $R^3$, $R^4$, $R^5$, $R^6$ or $R^7$ have up to 4, especially 1 or 2, carbon atoms. Preferably each of $R^3$, $R^4$, $R^5$, $R^6$ and $R^7$ independently represents a methyl group or a hydrogen atom. Preferably at least two of $R^4$, $R^5$, $R^6$ and $R^7$ are hydrogen.

Preferably m is 0.

The compounds of formula I are capable of forming acid addition salts. Such salts may be with organic or inorganic acids. For example they may be with sulphonic acids such as toluene or benzene sulphonic acid, with carboxylic acids such as acetic or oxalic acid, or with mineral acids, such as hydrohalic acids, especially hydrochloric acid, or sulphuric or nitric acid.

The compounds of formula I also form metal salt complexes. Suitable metals include copper, zinc, manganese and iron, and the anion may be derived from one of the acids listed above.

It will be appreciated that the compounds of formula I may contain asymmetric centres depending on the various groups present, for example at the saturated carbon atoms in the thiazinone ring, and thus that they may exist in the form of different isomers. Accordingly the present invention encompasses the individual isomers as well as racemic and other mixtures of isomers of these compounds.

The invention also provides a process for the preparation of a compound according to the invention, which comprises cyclising a compound of the general formula

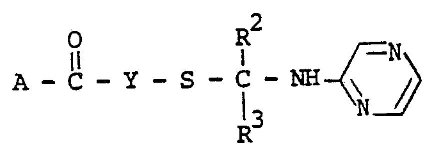

(II)

in which $R^2$, $R^3$ and Y have the meanings given above, and A represents an —OH group; and if desired, converting the resulting compound according to the invention into any other compound according to the invention.

The reaction is conveniently carried out in a solvent. Suitable solvents include hydrocarbons and chlorinated hydrocarbons, for example a benzenic solvent such as benzene, toluene, xylene or chlorobenzene, or an aliphatic solvent such as tetra-, tri- or di-chloromethane, or 1,2-dichloroethane. If desired, the water generated by the reaction may be distilled off continuously. In some cases, the use of a cyclizing or dehydrating agent, for example N,N-dicyclohexylcarbodiimide or N-ethoxycarbonyl-2-ethoxy-1,2-dihydroquinoline may be desirable, particularly when carrying out the reaction at relatively low temperatures. In general, the reaction may be carried out, for example, at a temperature of from 0 to 150°C. It is usually convenient to carry out the reaction either at room temperature or at the reflux temperature.

The derivatives of formula I in which m=1 or 2 may be prepared by oxidising the corresponding derivative in which m is less than 1 or 2 respectively. This may be carried out using conventional oxidising agents, for example peroxides such as hydrogen peroxide, or peracids such as metachloroperbenzoic acid or peracetic acid, or potassium permanganate. Conveniently the derivative to be oxidised is dissolved in a suitable solvent, for example a chlorinated hydrocarbon solvent such as chloroform or dichloromethane, or a carboxylic acid such as acetic acid.

The derivatives wherein $R^4$, $R^5$, $R^6$ or $R^7$ are not hydrogen can if desired be conveniently prepared by alkylation of corresponding hydrogen analogues by methods known in the art, such as those described in GB—A—2031 892.

The compounds of formula I may be prepared in the form of salts or may be converted into salts or complexes by methods known in the art, such as reaction with the appropriate acid or metal salt. Thus the basic compound may be dissolved in a suitable solvent, such as an ether, for example diethyl ether, and acid or metal salt added.

The compounds of formula I may be isolated from the reaction medium in known manner, and it will be appreciated that different isomeric forms can be isolated from one another by methods known in the art, for example by chromatography.

The compound of formula II may be prepared by reacting an imine of the general formula

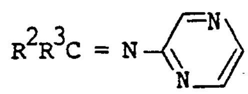

(III)

with a compound of the general formula

$$HS.Y.CO.A \qquad (IV)$$

where $R^2$, $R^3$ and Y have the meanings given above, and A represents an —OH group. Preferably the reaction is carried out in the presence of a solvent, for example one of those listed above for the cyclisation reaction, such as toluene, benzene, xylene or chlorobenzene. The reaction temperature is preferably in the range of from 0 to 150°C, for example at room temperature or at the reflux temperature.

In some cases, it may be convenient not to isolate the compound of the general formula II, but to carry out the reaction of the compounds III and IV under conditions such that cyclisation of the resulting compound II takes place *in situ.*

The compounds of formulae III and IV may be prepared by methods known in the art. Thus the imine may be prepared by reacting aminopyrazine with an aldehyde or ketone, $R^2R^3C{=}O$, conveniently in the presence of a solvent; the reaction may be carried out at reflux temperature and the water of the reaction distilled off continuously.

If desired, the preparation of the imine, and its reaction with a compound of formula IV can be carried out without isolation of the imine, for example by reacting the aldehyde or ketone, $R^2R^3C{=}O$, and aminopyrazine with the compound of formula IV, in the presence of a suitable solvent.

The compounds of formula I and their salts and metal salt complexes have been found to have fungicidal properties, and accordingly the invention further provides a fungicidal composition comprising a compound according to the invention in association with at least one carrier, and a method of making such a composition which comprises bringing a compound according to the invention into association with at least one carrier.

The invention also provides the use of a compound or composition according to the invention as a fungicide. Further in accordance with the invention there is provided a method of combating fungus at a locus, by treating the locus with a compound or composition according to the invention, and particularly a method of protecting crop plants from fungal attack comprising treating crop plants subject to or subjected to fungal attack, seeds of such crop plants or the medium in which such plants are growing or are to be grown with a compound or a composition according to the invention.

The compounds according to the invention are generally applied at a dosage rate of, for example, 0.025 to 10 kg/ha. In general, for application to plant foliage for prophylactic or therapeutic treatment preferred dosage rates will be in the range 0.1 to 1.0 kg/ha. For soil treatment appropriate rates of application may vary widely, for example 0.5 to 5.0 kg/ha. In use for seed treatment, preferred rates of application may be 1.0 to 10 g/kg of seed.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used. A composition according to the invention generally contains from 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example

kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example, a composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated caster oil, and sodium alkylaryl sulphonates such as sodium dodecyl-benzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50, and 75% w of active ingredient and usually contain, in addition to solid inert carrier, 3—10% w of a dispersing agent and, where necessary, 0—10% w of stabiliser(s) and/or other additives such as penetrants or stickers. Dusts are usually formulated as a dust concentrate having similar composition to that of a wettable powder but without a dispersant, and are diluted in the field with further solid carrier to give a composition usually containing $\frac{1}{2}$—10% w of active ingredient. Granules are usually prepared to have a size between 10 and 100 BS mesh (1.676—0.152 mm), and may be manufactured by agglomeration or impregnation techniques. Generally, granules will contain $\frac{1}{2}$—25% w active ingredient and 0—10% of additives such as stabilisers, slow release modifiers and binding agents. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqeuous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions may also contain other active ingredients, for example compounds having insecticidal, fungicidal or herbicidal properties. In particular, the use of compositions containing a second fungicidal compound may be advantageous, for example to broaden the spectrum of activity.

The invention is further illustrated by the following examples. In these examples the identity of the products was confirmed by NMR analysis.

### Example 1

2-(4'-chlorophenyl)-tetrahydro-3-pyrazinyl-4H-1,3-thaizin-4-one

A mixture of aminopyrazine (19g, 0.2 mol) and 4-chlorobenzaldehyde (28.1g, 0.3 mol; in toluene (250 ml) was stirred and refluxed under a Dean and Stark trap for 16 hours. After cooling, 3-mercaptopropionic acid (23.3g, 0.22 mol) dissolved in a little toluene was added. The solid resulting was filtered off, washed with toluene and then with petroleum spirit, and dried. 10g of this solid was dissolved in methylene chloride (250ml), N,N'-dicyclohexylcarbodiimide (10g) was added, and the mixture was stirred at room temperature for 12 days. The mixture was then filtered, and the solvent removed from the filtrate *in vacuo.* The residue was purified by chromatography over silica gel, eluting with diethyl ether, and then by further chromatography over silica gel, eluting with 10% diethyl ether in methylene chloride. The desired compound was obtained in 20% yield, melting point 112.5—114.5°C.

| | | |
|---|---|---|
| Analysis | $N_3OSClC_{14}H_{12}$ | Theory 54.99% C 3.93% H 13.75% N |
| | | Found 55.2% C 3.9% H 13.6% N |

### Examples 2 to 7

The following compounds were prepared according to methods similar to those of the above Example 1. The melting points and chemical analysis of the compounds of Examples 2 to 7 are given in Table I below.

TABLE I

| Example No. | In the General Formula I $R^2$ | $R^3$ | Y | m | Melting Point °C | Analysis Theory C | H | N | Found C | H | N |
|---|---|---|---|---|---|---|---|---|---|---|---|
| 2 | 4-chloro-phenyl | H | $C(CH_3)_2CH_2$ ($CH_2$ adjacent the S atom) | 0 | 119—120 | 57.57 | 4.80 | 12.59 | 58.3 | 4.8 | 12.8 |
| 3 | 2,4-dichloro phenyl | H | $CH_2CH_2$ | 0 | 153—156 | 49.41 | 3.24 | 12.35 | 49.7 | 3.5 | 12.4 |
| 4 | 2,4-dichloro phenyl | H | $C(CH_3)_2CH_2$ ($CH_2$ adjacent the S atom) | 0 | 213—217 | 52.17 | 4.08 | 11.41 | 51.3 | 4.1 | 11.3 |
| 5 | 2,4-dichloro phenyl | H | $C(CH_3)_2CH_2$ ($CH_2$ adjacent the S atom) | 1 | 176—178 | 47.19 | 3.09 | 11.80 | 47.3 | 3.0 | 11.8 |
| 6 | 2,4-dichloro phenyl | H | $C(CH_3)_2CH_2$ ($CH_2$ adjacent the S atom) | 2 | 179—181 | 45.16 | 2.96 | 11.29 | 45.3 | 3.0 | 11.3 |
| 7* | 2,4-dichloro phenyl | H | $C(CH_3)_2CH_2$ ($CH_2$ adjacent the S atom) | 0 | 154—156 | 44.62 | 3.19 | 11.16 | 44.7 | 3.1 | 10.9 |

* Hydrochloride salt of the free base of formula I.

Example 8

The activity of compounds according to the invention was determined by the following test procedures. In all cases, the test solution was obtained by diluting with water to give the required dosage, a stock solution containing the active material in acetone (50%), surfactant (0.4%) and water.

(a) Activity against apple powdery mildew (Podosphaera leucotricha; P.1)

This test is a direct anti-sporulant one using a foliar spray. The upper surfaces of leaves of whole apple seedlings were inoculated by spraying with an aqueous suspension containing $10^5$ conidia/ml 2 days prior to treatment with the test compound. The inoculated plants were immediately dried and kept at glasshouse ambient temperatures and humidity prior to treatment. The plants were sprayed at a dosage of 1 kilogram of active material per hectare using a track sprayer. After drying the plants were returned to a compartment at ambient temperature and humidity for up to 9 days, followed by assessment. Assessment was based on the percentage of the leaf area covered by sporulation compared with that on leaves of control plants.

(b) Activity against potato/tomato late blight (Phytophthora infestans; P.i.p.)

The test measures the direct protectant activity of compounds applied as a foliar spray. Tomato plants, Cultivar Ailsa Craig, 1—15 cms high, were used. The whole plant was sprayed at a dosage of 1 kilogram of active material per hectare using a track sprayer. The plant was then inoculated up to 6 hours after treatment with the test compound, by spraying with an aqueous suspension containing 5 $\times 10^3$ zoospores/ml. The inoculated plants were kept in high humidity for 3 days. Assessment was based on comparison between the levels of disease on the treated and control plants.

(c) Activity against barley powdery mildew (Erysiphe graminis; Eg.)

The test measures the direct anti-sporulant activity of compounds applied as a foliar spray. For each compound about 40 barley seedlings were grown to the one-leaf stage in a plastic pot of sterile potting compost. Inoculation was effected by dusting the leaves with conidia of Erysiphe graminis, spp. hordei. 24 hours after inoculation the seedlings were sprayed at a dosage equivalent to 1 kg of active material per hectare. First assessment of disease was made 5 days after treatment, when the overall level of sporulation on the treated plants were compared with that on control plants.

(d) Activity against barley powdery mildew (Erysiphe graminis; Eg (soil))

The test measures the systemic protectant activity of compounds applied as a soil drench. For each compound, soil was drenched at a dosage equivalent to 10kg/ha. About 25 barley seeds, cv. Golden Promise, were placed on top of the treated soil, and covered with about 1cm untreated soil. After about 7 days when the seeds had germinated, spores of the fungus were dusted onto the plants. After 5 days, the level of sporulation was compared with that on control plants.

(e) Activity against vine grey mould (Botrytis cinera; B.c)

The test is a direct eradicant one using a foliar spray. The under-surface of detached vine leaves were inoculated by pipetting ten large drops of an aqueous suspension containing $5 \times 10^5$ conidia/ml on to them. The inoculated leaves were kept uncovered overnight during which time the fungus penetrated the leaf and a visible necrotic lesion became apparent where the drop was made. The infected regions were sprayed directly with a dosage of 1 kg of active material per hectare using a track sprayer. When the spray had dried the leaves were covered with petri dish lids and the disease allowed to develop under the moist conditions. The extent of the necrotic lesion beyond the original drop together with the dosage of sporulation was compared with that on control leaves.

(f) Activity against peanut leaf spot (Cercospora arachidicola; C.a)

The test is a direct eradicant one using a foliar spray. The upper surfaces of the leaves of peanut plants (12—20 cms high, in monopots) where inoculated by spraying with an aqueous suspension containing $10^5$ conidia/ml 40—43 hours prior to treatment with the test compound. The inoculated plants were kept at high himidity and then allowed to dry during the interval between inoculation and treatment by spraying at a dosage of 1kg of active material per hectare using a track sprayer. After spraying the plants were moved to a humid compartment at 25—28°C for a further period of up to 10 days. Assessment was based on a comparison between the levels of disease on the treated and control plants.

The extent of disease control achieved in these tests is expressed as a control rating in which disease control greater than 80% is given the rating 2, and control of between 50 and 80% is given the rating 1. The results are given in Table II below.

TABLE II TEST RESULTS

| Compound of Example No. | Test Result | | | | | |
|---|---|---|---|---|---|---|
| | P.i.p | Eg | Eg(soil) | P.I. | B.c. | C.a. |
| 1 | | 2 | 2 | 2 | 2 | |
| 2 | | 2 | 2 | | | |
| 3 | 1 | 2 | 1 | 2 | 2 | |
| 4 | | 2 | 2 | 2 | 2 | |

**Claims**

1. A compound of the general formula

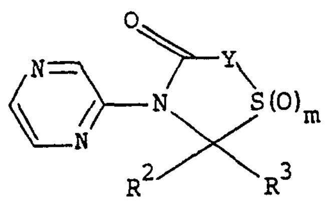

(I)

or a salt or a metal complex thereof, wherein Y represents a group $—CR^4R^5CR^6R^7—$; $R^2$ represents a phenyl group substituted by one or two halogen atoms; each of $R^3$, $R^4$, $R^5$, $R^6$ and $R^7$ independently represents a hydrogen atom or a $C_{(1-4)}$ alkyl group; and m is 0, 1 or 2.

2. A compound as claimed in claim 1, in which each of $R^3$, $R^4$, $R^5$, $R^6$ and $R^7$ independently represents a methyl group.or a hydrogen atom.

3. A compound as claimed in claim 2, in which at least two of $R^4$, $R^5$, $R^6$ and $R^7$ represent hydrogen atoms.

4. A compound as claimed in any one of claims 1 to 3 in which m is 0.

5. Any of the following compounds:
2-(4'-chlorophenyl)-tetrahydro-3-pyrazinyl-4*H*-1,3-thiazin-4-one;
2-(2',4'-dichlorophenyl)-tetrahydro-3-pyrazinyl-4*H*-1,3-thaizin-4-one; or
2-(2',4'-dichlorophenyl)-tetrahydro-5,5-dimethylpyrazinyl-4*H*-1,3-thiazin-4-one; or the hydrochloride salt or the 1-oxide or 1,1-dioxide thereof.

6. A process for the preparation of a compound as claimed in claim 1, which comprises cyclising a compound of the general formula

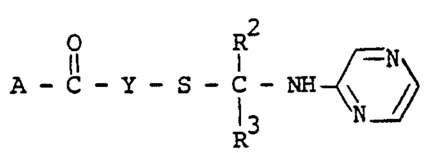

(II)

in which $R^2$, $R^3$ and Y have the meanings given in claim 1, and A represents an —OH group; and if desired, converting the resulting compound according to the invention into any other compound according to the invention.

7. A process as claimed in claim 6, in which the compound of the general formula II has been prepared by the reaction of a compound of the general formula

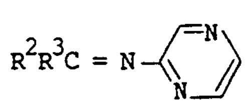

(III)

with a compound of the general formula

HS.Y.CO.A. (IV)

where $R^2$, $R^3$, Y and A have the meanings given in claim 6.

8. A fungicidal composition which comprises a compound as claimed in any one of claims 1 to 5, together with a carrier.

9. A composition as claimed in claim 8, which comprises at least two carriers, at least one of which is a surface-active agent.

10. A method of combating fungal growth at a locus, which comprises treating the locus with a compound as claimed in any one of claims 1 to 5 or a composition as claimed in either claim 8 or claim 9.

**Revendications**

1. Composé de formule générale

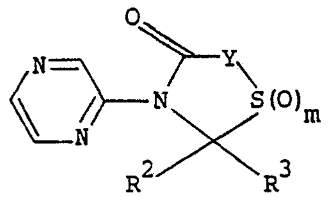

(I)

ou un de ses sels ou complexe métallifère, dans laquelle Y représente un groupe —$CR^4R^5CR^6R^7$—; $R^2$ représente un groupe phényle substitué par un ou deux atomes d'halogène; chaque $R^3$, $R^4$, $R^5$, $R^6$ et $R^7$ représente indépendamment un atome d'hydrogène ou un groupe alkyle $C_{(1-4)}$; et m est 0, 1 ou 2.

2. Composé selon la revendication 1, dans lequel chaque $R^3$, $R^4$, $R^5$, $R^6$ et $R^7$ indépendamment représente un groupe méthyle ou un atome d'hydrogène.

3. Composé selon la revendication 2, dans lequel au moins deux des groupes $R^4$, $R^5$, $R^6$ et $R^7$ représentent des atomes d'hydrogène.

4. Composé selon l'une quelconque des revendications 1 à 3 dans lequel m est 0.

5. L'un quelconque des composés suivants:

2-(4'-chlorophényl)-tétrahydro-3-pyrazinyl-4*H*-1,3-thiazinone-4

2-(2',4'-dichlorophényl)-tétrahydro-3-pyrazinyl-4*H*-1,3-thiazinone-4; ou

2-(2',4'-dichlorophényl)-tétrahydro-5,5-diméthylpyrazinyl-4*H*-1,3-thiazinone-4;

on leur sel chlorhydrate ou leur oxyde-1 ou leur dioxyde-1,1.

6. Procédé de préparation d'un composé selon la revendication 1, qui comprend la cyclisation d'un composé de formule générale

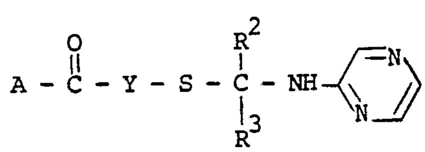

(II)

dans laquelle $R^2$, $R^3$ et Y ont les significations données dans la revendication 1, et A représente un groupe —OH; et si c'est souhaité, la conversion du composé résultant selon l'invention en l'un quelconque des autres produits selon l'invention.

7. Procédé selon la revendication 6, dans lequel le composé de formule générale II a été préparé par la réaction d'un composé de formule générale

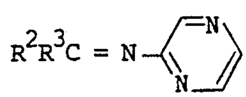

(III)

avec un composé de formule générale

HS.Y.CO.A (IV)

dans lesquelles $R^2$, $R^3$, Y et A ont les significations données dans la revendication 6.

8. Composition fongicide qui comprend un composé selon l'une quelconque des revendications 1 à 5, accompagné d'un véhicule.

9. Composition selon la revendication 8, qui comprend au moins deux véhicules, dont au moins l'un est un agent tensioactif.

10. Procédé pour combattre la croissance fongique sur un substrat, qui comprend de traiter le substrat avec un composé selon l'une quelconque des revendications 1 à 5 ou une composition selon soit la revendication 8, soit la revendication 9.

**Patentansprüche**

1. Eine Verbindung der allgemeinen Formel

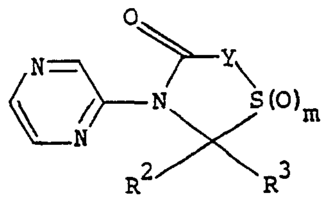

(I)

oder ein Salz oder Metallkomplex davon, in welcher Y eine Gruppe —$CR^4R^5CR^6R^7$— darstellt; $R^2$ eine Phenylgruppe, substituiert durch ein oder zwei Halogenatome, bedeutet; jede der Gruppen $R^3$, $R^4$, $R^5$, $R^6$ und $R^7$ unabhängig ein Wasserstoffatom oder eine $C_{(1-4)}$-Alkylgruppe ist; und m den Wert 0, 1 oder 2 hat.

2. Eine Verbindung wie in Anspruch 1 beansprucht, in welcher jede der Gruppen $R^3$, $R^4$, $R^5$, $R^6$ und $R^7$ unabhängig eine Methylgruppe oder eine Wasserstoffatom bedeutet.

3. Eine Verbindung wie in Anspruch 2 beansprucht, in welcher mindestens zwei der Gruppen $R^4$, $R^5$, $R^6$ und $R^7$ Wasserstoffatome darstellen.

4. Eine Verbindung wie in einem der Ansprüche 1 bis 3 beansprucht, in welcher m den Wert 0 hat.

5. Irgendeine der folgenden Verbindungen:
2-(4'-Chlorphenyl)-tetrahydro-3-pyrazinyl-4*H*-1,3-thiazin-4-on;
2-(2',4'-Dichlorphenyl)-tetrahydro-3-pyrazinyl-4*H*-1,3-thiazin-4-on); oder
2-(2',4'-Dichlorphenyl)-tetrahydro-5,5-dimethylpyrazinyl-4*H*-1,3-thiazin-4-on; oder das Hydrochloridsalz oder das 1-Oxid oder 1,1-Dioxid davon.

6. Ein Verfahren zur Herstellung einer Verbindung wie in Anspruch 1 beansprucht, welches das Cyklisieren einer Verbindung der allgemeinen Formel

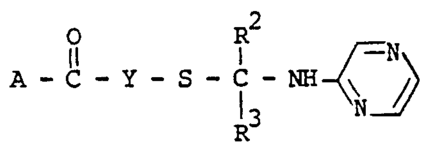

(II)

in welcher $R^2$, $R^3$ und Y die in Anspruch 1 angegebenen Bedeutungen haben, und A eine —OH-Gruppe darstellt; und, falls erwünscht, das Umwandeln der resultierenden Verbindung gemäß der Erfindung in eine andere Verbindung gemäß der Erfindungumfaßt.

7. Ein Verfahren wie in Anspruch 6 beansprucht, in welchem die Verbindung der allgemeinen Formel II hergestellt wurde durch Umsetzung einer Verbindung der allgemeinen Formel

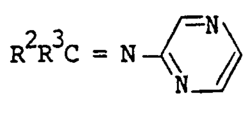

(III)

mit einer Verbindung der allgemeinen Formel

HS.Y.CO.A. (IV)

in welchen $R^2$, $R^3$, Y und A die in Anspruch 6 angegebenen Bedeutungen haben.

8. Eine fungizide Zusammensetzung, welche eine Verbindung, wie in einem der Ansprüche 1 bis 5 beansprucht, zusammen mit einem Träger enthält.

9. Eine Zusammensetzung wie in Anspruch 8 beansprucht, welche mindestens zwei Träger umfaßt, wovon mindestens einer eine oberflächenaktive Substanz ist.

10. Ein Verfahren zur örtlichen Bekämpfung von Pilzwachstum, welches das Behandeln der betroffenen Stelle mit einer Verbindung, wie in einem der Ansprüche 1 bis 5 beansprucht, oder einer Zusammensetzung wie in Anspruch 8 oder Anspruch 9 beansprucht, umfaßt.